# EUROPEAN PATENT SPECIFICATION

(11) **EP 2 286 712 B1**
(45) Date of publication and mention of the grant of the patent: **17.08.2016**
(21) Application number: 10170969.9
(22) Date of filing: 27.07.2010
(51) Int. Cl.: A47L 23/20, A61L 2/20

(54) **Boot and glove dryer for food service industry, and method of making same**
Schuh- und Handschuhtrockner für die Lebensmittelindustrie, und Herstellungsverfahren dafür
Séchoir pour bottes et gants pour l'industrie des services alimentaires, et son procédé de fabrication

(30) Priority: 19.08.2009 US 235129 P; 10.06.2010 US 797648
(43) Date of publication of application: 23.02.2011
(73) Proprietor: Williams Boot & Glove Dryers Inc., Langley, British Columbia BC V3A 4N4 (CA)
(72) Inventor: Williams, Gary, Langley, British Columbia BC V2Z2C3 (CA)
(74) Representative: Copsey, Timothy Graham

(56) References cited:
- EP-A1- 0 479 241
- EP-A1- 0 516 563
- EP-A1- 1 369 076

## Description

### BACKGROUND

The present disclosure relates to forced air dryer systems for rack drying boots and gloves, and particularly, to such devices in which the rack is either wall mounted or free standing and readily moveable for simultaneous drying of several pairs of boots and/or gloves from a common blower system. Such forced air type dryers are often employed for drying boots utilized in the construction industries and/or boots and gloves for uniformed emergency responders such as for hazardous waste disposal and fire fighters. Upon removal, the user may hang the boots and/or gloves on the rack and the forced air system circulates air to the interior of the gloves and/or boots and, over a period of a few hours, the interior is completely dried. If accelerated drying is required, a heater may be employed with the blower to circulate heated air interiorly of the gloves and/or boots.

In the food service industry including food processing, where workers are required to wear sterilized boots and gloves, it is required that the boots and gloves be sprayed with liquid sterilizing or disinfecting agent, such as bleach, prior to drying. The presence of the liquid sterilizer must be controlled such that pooling or residual amounts of liquid sterilizer are not retained on any horizontal surface such that bacteria could be collected over a time interval prior to total evaporation or drying of the sterilization liquid. In this regard, it has been found beneficial to provide for rack mounting of the boots/gloves to optimize the sterilization process and prevent contamination.

EP1369076 to Marolt discloses a shoe dryer with a tubular support through which heated air is supplied directly to the inside of the shoe. EP0516563 to Deroux relates to a process for shoe-washing and disinfecting. The shoes are placed in a position which allows water to flow out easily and are then subjected to cycles of washing and disinfection under various pressures. EP0479241 to Kimura describes a drying apparatus for rubber boots and other articles. Forced air convection is used to carry liquid disinfectant on an air stream into compartments containing the articles to be dried.

Accordingly, it has been desired to provide a way or means of air drying boots and/or gloves requiring sterilization with forced air circulation interiorly thereof in a manner and with equipment that prevents liquid pooling and completely drains of any sterilizer. It has been particularly desired to provide a system in a wall rack version and a free standing portable rack version capable of drying a multiple number of pairs of boots and/or gloves which require sterilization without trapping any of the sterilizing liquid exteriorly on or interiorly of the air drying system.

### BRIEF DESCRIPTION

According to the present invention, there is provided a forced air drying system for boots and/or gloves for use in food processing requiring spraying with sterilizer/disinfectant; with a rack with a plurality of spaced tubular members adapted for receiving thereon the boots and/or gloves to be dried;
a manifold connected to the tubular members; and a blower housing mounted on the manifold, the housing including an air inlet, wherein any horizontal exterior surfaces of the housing, manifold and tubular members are angled or sloped to prevent pooling of the sterilizer/disinfectant.

The present disclosure describes a system including a rack for receiving multiple pairs of boots and/or gloves with a blower for circulating air interiorly of the gloves and/or boots for effecting interior drying thereof in which the boots and/or gloves are subject to sterilization with a sterilizing agent sprayed thereon when mounted on the rack. The blower system and the hollow members of the rack are configured such that there is a complete absence of exterior horizontal surfaces and that pooling of the sterilizing agent does not occur on any non-vertical interior or exterior surfaces. In addition, the interior surfaces of the distribution tubes are provided with strategically located drain holes such that the sterilizing agent or moisture from condensation, upon entering the interior of any of the blower housing, air distribution manifold, distribution tubes or boot/glove holding tubes, is completely drained to prevent trapping or pooling of the sterilizing agent. One disclosed version is intended for sterilizing with electrical power connected; and, another version is disclosed which is intended for sterilizing only after electrical power has been externally switched off. Both versions are adapted for wall mounting or alternatively may be arranged as free standing portable single or double sided rack drying systems. Wall mounting may be achieved through the use of a wall mounting bracket or other suitable means.

The blower housing may include a fluid drain arranged to drain into the manifold. The manifold may drain into the tubular members of the rack. The present disclosure also relates to a method of making a forced air drying system for boots and/or gloves for use in food processing requiring spraying with sterilizer or disinfectant. The method comprises providing a rack with a plurality of spaced tubular members adapted for receiving thereon the boots and/or gloves to be dried, providing an air manifold and connecting the manifold to the tubular members, and disposing a blower housing on the manifold and sloping all exterior horizontal surfaces on the manifold and blower housing in order to prevent the pooling of sterilizer or disinfectant.

### BRIEF DESCRIPTION OF THE DRAWINGS

FIGURE 1 is a perspective view from the front of a wall mounted version of the forced air drying system and rack for multiple pairs of boots and/or gloves according to the present disclosure;
FIGURE 2 is a perspective view from the back of the version of FIGURE 1;
FIGURE 3 is an enlarged view of FIGURE 2 with portions broken away to illustrate the interior of the blower housing and air distribution manifold of the system of FIGURE 1;
FIGURE 4 is a perspective view of a free standing single sided rack portable version of the forced air drying system;
FIGURE 5 is a perspective view of a free standing double sided rack portable version of the forced air drying system;
FIGURE 6 is a section view taken along section-indicating lines 6-6 of FIGURE 1;
FIGURE 7 is a section view taken along section-indicating lines 7-7 of FIGURE 3;
FIGURE 8 is an enlarged view of the lower end portion of a distribution tube of FIGURE 1; and
FIGURE 9 is a portion of a view similar to FIGURE 1 showing another version with a louvered enclosed blower housing.

### DETAILED DESCRIPTION

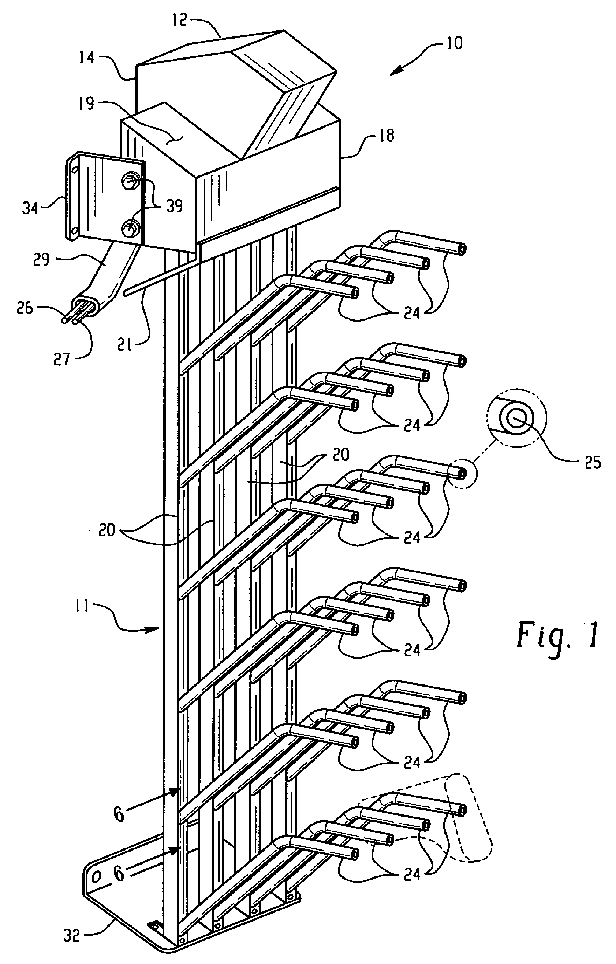

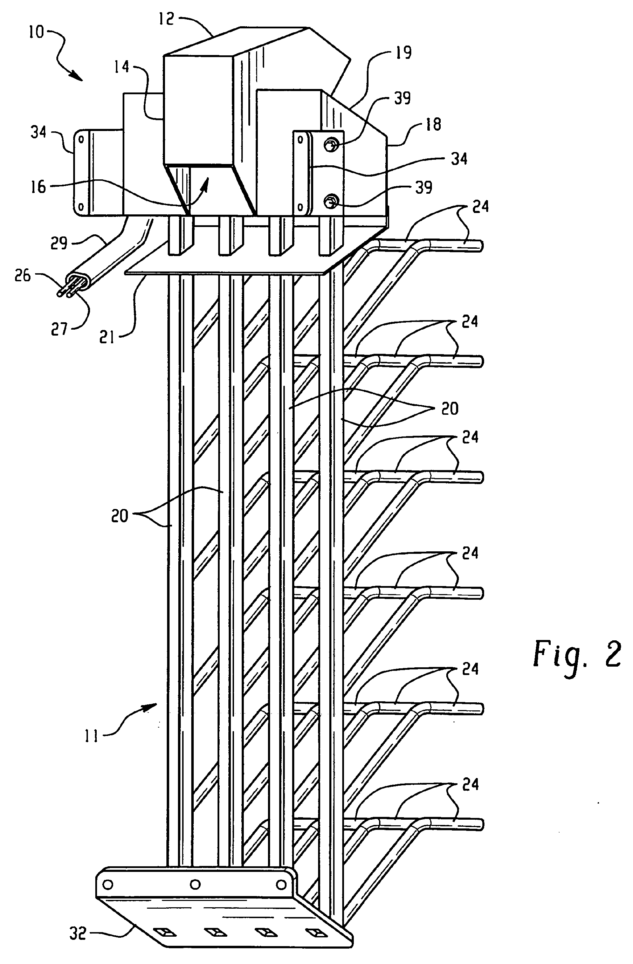

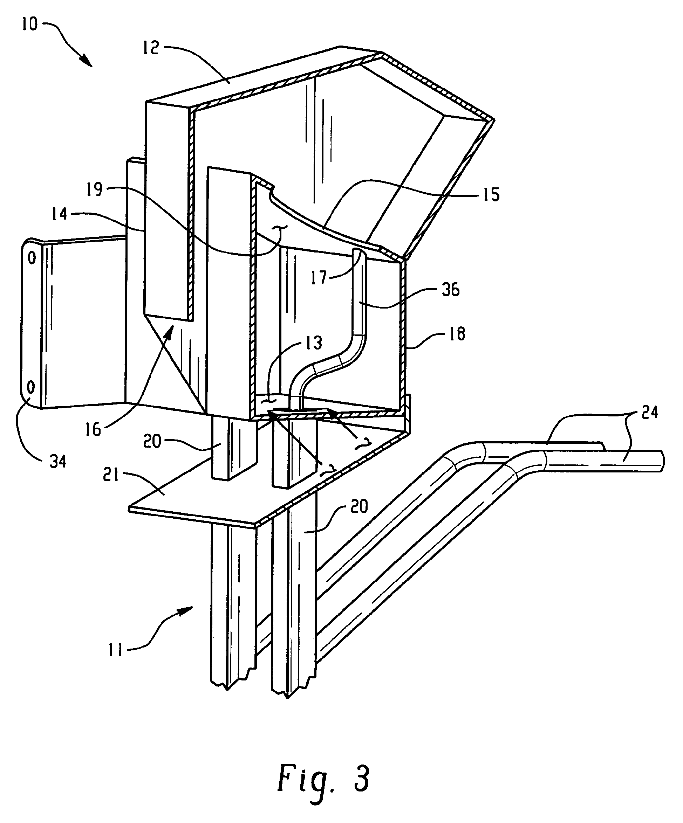

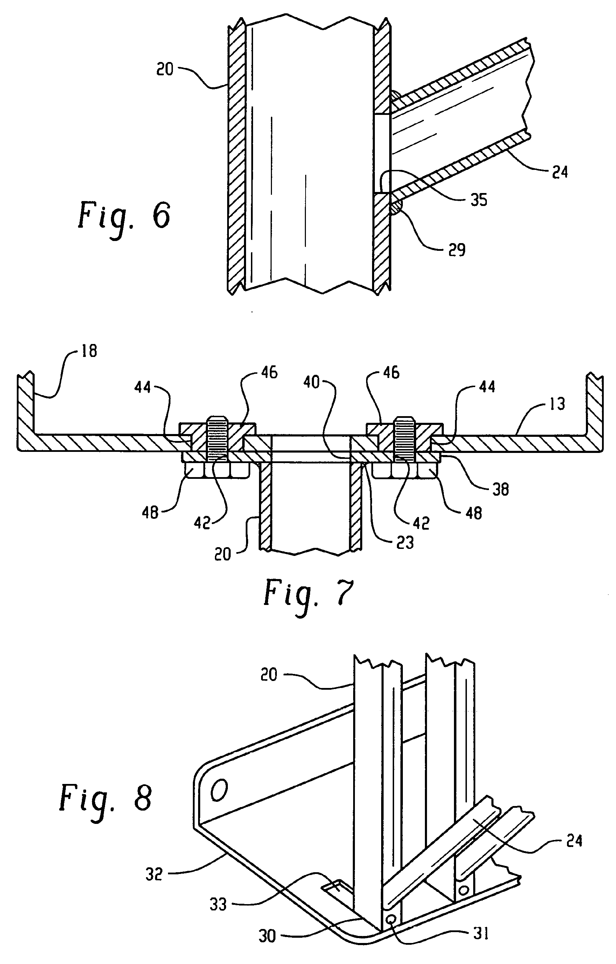

Referring to FIGURE 1, a forced air drying system for drying the interior of multiple pairs of boots or gloves is shown generally at 10 as adapted to be mounted on a wall or vertical surface panel for accommodating multiple pairs of boots and/or gloves for simultaneous drying. The system 10 includes a blower housing 12 having, disposed interiorly thereof, a suitable blower (not shown); and, the housing 12 has a portion thereof overhanging and extending downwardly along the back side of an air distribution manifold 18. If desired, an unshown heater may be incorporated within the blower housing 12. Blower housing 12 sits atop a sloping upper surface 19 of the air distribution manifold 18; and, the surface 19 has openings therein such as 15 (see FIGURE 3) for enabling forced air from the blower to enter the manifold. As shown in FIGURES 2 and 3, the lower end of an overhanging portion 14 of the blower housing is open and forms the air inlet indicated generally at 16 for the blower housing. The air inlet 16 may include an air passage on the blower housing (not shown). Manifold 18 has a rack indicated generally at 11 with a plurality of distribution tubes 20 connected to the underside of the floor 13 of manifold 18, which tubes 20 extend downwardly therefrom in spaced generally parallel arrangement. As shown typically in FIGURE 3 and FIGURE 7, the interior of each of the distribution tubes 20 is open to the interior of the manifold 18 permitting air in the manifold to enter each of the distribution tubes 20. Tubes 20 may be attached to the manifold by any suitable expedient such as shown in FIGURE 7, wherein a flange 38 is attached to the end of distribution tube 20, such as for example by weldment, the flange having aperture 40 conforming to the interior of tube 20. The flange 38 has a plurality of fastener receiving apertures 42 disposed about aperture 40. The manifold floor 13 has correspondingly located apertures 44 into which are pressed threaded nuts 46 such as for example T-nuts. If desired, the T-nuts may be additionally secured such as by staking or weldment. The tubes are then secured by threaded fasteners 48 engaging nuts 46 through apertures 42 in flange 38.

As shown in FIGURE 3, the interior of the manifold has at least one discrete drain conduit in the form of tube 36 vertically oriented such that its upper end communicates with a drain hole 17 formed in a sloping surface 19 of the manifold; and, tube 36 is configured such that the lower end of drain tube 36 is oriented to discharge into one of the distribution tubes 20 for draining the blower housing directly into the distribution tubes. In operation, as air is forced into the manifold and down the tubes 20, an aspirating effect is created on the lower end of tube 36, thus facilitating draining of any liquid in the blower housing 12.

FIGURES 1 and 3 illustrate further details of the system 10 which has the blower housing disposed such that the upper surface thereof is sloped thereby providing a housing with no horizontal surfaces which could trap or pool sterilizer or disinfectant sprayed over the system. As shown in FIGURES 1-3, the manifold may have an optional splash guard 21 to prevent sprayed sterilizer or disinfectant from entering the air inlet attached thereto and angled downwardly under the manifold.

The version of FIGURES 1-3 is thus intended to protect the blower system from liquid entry where electrical power is being supplied to the blower during spraying of liquid sterilizer/disinfectant.

Each of the distribution tubes 20 has at least one, and preferably a plurality, of boot/glove hanging tubes extending outwardly therefrom in cantilever as denoted by reference numerals 24 in FIGURES 1-3. Each of the cantilevered tubes may include a removable airflow choke disposed in the free end thereof. In the present practice, it has been convenient to have the distribution tubes 20 formed with a rectangular or square cross section to facilitate attachment of the hanging tubes 24. In one exemplary version of the present forced air drying system, it has been found satisfactory to form the distribution tubes 20 of square tubing having a side width of 38 mm (1.5 inches) and the hanging tubes 24 of 25 mm (1.0 inch) diameter round tubing;
however, other cross sections and sizes may be employed. As shown typically in FIGURES 3 and 6, each of the tubes 24 has one end thereof attached to one of the distribution tubes 20; and, each of the tubes 24 has the interior thereof open to the interior of the distribution tube 20 through an orifice 35 formed in the wall of the distribution tube 20, to provide fluid communication therewith such that air forced into the tubes 20 is also forced into the tubes 24 and out through the open end of each of the tubes 24. Tubes 24 may be attached to the tubes 20 by any suitable expedient such as by weldment 29 shown in FIGURE 6.

Referring to the enlarged encircled view in FIGURE 1, in the present practice, it has been found desirable to provide an appropriately sized air flow choke orifice 25 in the end of each hanging tube 24 to balance airflow throughout the rack. The choke orifices may be conveniently formed in plastic inserts provided in the ends of the tubes 24. The plastic inserts forming the orifices 25 may be configured for ready removal and replacement as may be required to provide additional protection against contamination. It will be understood that the open end of a boot is received for drying over the tube with the toe portion of the boot hanging downwardly as illustrated in dashed outline in FIGURE 1.

Referring to FIGURES 1 and 2, the unshown blower disposed within the housing 12 is adapted for connection to a source of electrical power by suitable electrical leads such as leads 26, 27 encased in suitable insulation to form a power cord 29. For food processing applications of the system of FIGURE 1 in which sterilization is required, it will be understood that the connection of power cord 29 to the manifold, blower housing and blower must be sealed against liquid penetration, which sealing may be accomplished in any convenient known manner. The system of FIGURES 1 and 2 is thus intended for operation where the sterilizing will be performed with electrical power connected to the system; and, if desired, while the blower is in operation.

Referring to FIGURE 8, one of the vertical distribution tubes 20 is shown with one of the boot/glove hanging tubes 24 attached thereto near the lower end of the distribution tube 20. FIGURE 8 illustrates the angled closed end 30 of the tube 20 such that any liquid entering the tube 20 drains to the lowest point of the angled surface. Drain holes 31 are provided at the lowest point of the surface of each tube 20 to drain any liquid entering the distribution tube 20 from the manifold or from the tubes 24. Cut outs 33, which may be punched in a bracket 32, prevent trapping or buildup of liquid behind distribution tubes 20.

Referring to FIGURE 1, the system 10 as shown is adapted to be anchored to a wall or vertical panel at the lower end of the tubes 20 by the bracket 32 and by at least one bracket 34 at the manifold 18. In the present practice, it has been found satisfactory to provide wall mounting surfaces such as a bracket 34 attached on each opposite side of the manifold 18 as shown in FIGURE 2 for example by bolts 39 to the wall of the manifold. The bracket for supporting the distribution tubes can be remote from the manifold. Alternatively, the wall mounting surfaces may be integrally formed with the manifold. Although the system 10 has been shown mounted on a wall, alternatively, it may be mounted to a free standing pedestal if desired, as will be hereinafter described. In the present practice, it has been found satisfactory to form the blower housing 12, manifold 18, distribution tubes 20 and hanging tubes 24 of stainless steel, however, other corrosion resistant materials could be used.

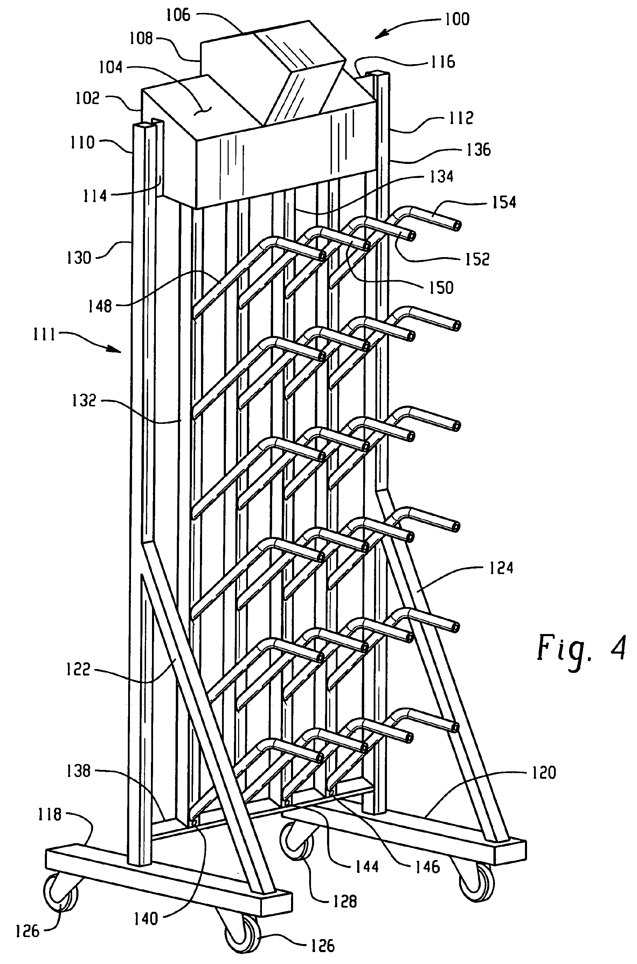

Referring to FIGURE 4, another version of the forced air drying system is indicated generally at 100 and has a manifold 102 with sloping upper surface 104 having a blower housing 106 mounted thereon with a rearward overhanging portion 108 provided thereon for forming an air inlet (not shown) in a manner similar to air inlet 16 of the FIGURE 1 version. Manifold 102 is supported on opposite sides thereof by posts 110, 112 which may comprise tubular members or channels and which may be attached to the manifold by attachment brackets 114, 116 by any suitable expedients such as weldment or fasteners. It will be understood that sloping upper surface 104 of the manifold 102 is provided with an unshown drain similar to drain 17 and also provided with an air passage similar to passage 15.

Posts 110, 112 are supported at their lower end on horizontal support or beam members 118, 120 and maintained positioned vertically by diagonal braces 122, 124, respectively. In the present practice, it has been satisfactory to form the posts, braces and horizontal beam members of tubular configuration to minimize the weight of the assembly. Horizontal beam members 118, 120 upon which posts 110, 112 are supported may each have rollers 126, 128, respectively, mounted on the undersurface thereof at opposite ends to permit the entire assembly to be rolled about and thus rendered portable. If desired, at least a pair of the rollers may be swiveled or castored.

The manifold 102 has attached to the undersurface thereof and extending downwardly therefrom a rack indicated generally at 111 which includes a plurality of spaced generally parallel tubular members 130, 132, 134, 136. The manifold has apertures formed therein (unshown) such that the tubular members 130, 132, 134, 136 each have the interior thereof fluidically communicating with the interior of manifold 102 in a manner similar to that of FIGURES 3 and 7. The lower end of each of the distribution tubes 130, 132, 134, 136 is sloped and has a sloped cross-member 138 attached thereto for closing the lower end of the distribution tubes 130, 132, 134, 136. Drain holes 140, 144, 146 are provided in the lower edge of each of the distribution tubes 130, 132, 134, 136 for draining moisture therefrom. Each of the distribution tubes 130, 132, 134, 136 has provided thereon extending generally upwardly and outwardly in cantilevered arrangement a plurality of hanging tubes denoted 148, 150, 152, 154, respectively. Each of the hanging tubes has an appropriately sized choke orifice provided in the free end thereof sized to equalize the flow of air throughout the distribution tubes and hanging tubes. In the present practice it has been found satisfactory to form the choke orifices in removable and replaceable inserts, which may be formed of plastic material provided in the ends of the hanging tubes. In the present practice, it has been convenient to form the manifold, blower housing, posts, distribution tubes, hanging tubes and structural support members of stainless steel or other corrosion resistant material. The attachment of the hanging tubes to the distribution tubes and the attachment of the distribution tubes to the manifold may be accomplished by any suitable expedient; for example, by welding or brazing.

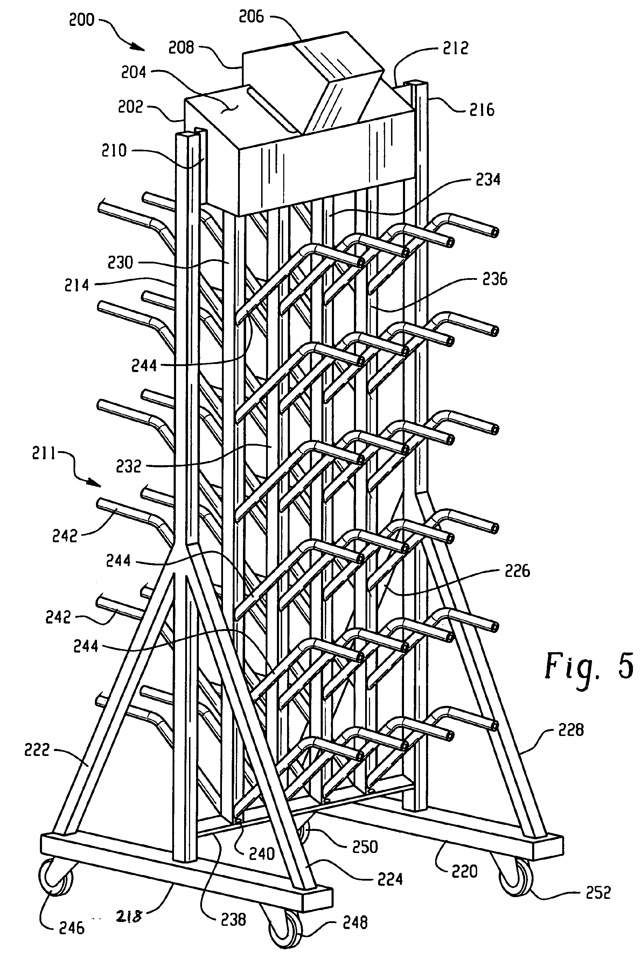

Referring to FIGURE 5, another version of a free standing portable drying system in accordance with the present disclosure is indicated generally at 200 and has a manifold 202 with sloping upper surface 204 having mounted thereon a blower housing 206. Housing 206 has a rearward overhanging portion 208 which forms an air inlet (not shown); and, housing 206 has enclosed therein an unshown blower which may include a heater, if desired. Manifold 202 has attached to the opposite sides thereof mounting brackets 210, 212, each of which is attached to and supported by a vertical post 214, 216, respectively, which posts may conveniently be formed of square tubing. Posts 214, 216 are each supported at its lower ends by horizontal support members in the form of beams 218, 220, respectively; and, diagonal braces 222, 224, 226, 228 are provided to position and maintain the posts vertically aligned. The posts, braces and horizontal beam members may be conveniently formed of tubular or channel stock for minimizing weight.

The manifold 202 has extending downwardly from the under surface thereof, a plurality of spaced generally parallel distribution tubes 230, 232, 234, 236 which have the lower ends thereof sloped and attached to a sloped cross-member 238 which closes the lower end of each of the distribution tubes and is attached at its opposite ends to the posts 214, 216, respectively. Each of the distribution tubes 230, 232, 234, 236 has a drain hole, such as hole 240 in tube 230, at its lower edge for draining any interior moisture.

Each of the distribution tubes 230, 232 has a plurality of hanging tubes 242 connected thereto and extending in cantilever therefrom on a common face thereof; and, each of the distribution tubes 230, 232 also has a second plurality of hanging tubes 244 connected thereto and extending in cantilever therefrom on the opposite side of the distribution tubes 230, 232 to thereby form a double sided rack 211 for hanging boots and gloves thereon for drying. Each of the hanging tubes 244 has an airflow choke orifice provided in the end thereof in a manner similar to that of the embodiment of FIGURE 1.

The drying system may comprise a support structure connected to the manifold and rack for maintaining the rack in a free standing position. The supporting structure, shown in the form of horizontal beam members 218, 220 may be optionally provided on the opposite ends thereof with rollers 246, 248 and 250, 252 which may be swiveled or castored if desired, to permit portability of the assembly 200. In the present practice, it has been found satisfactory to form the manifold, blower, housing, distribution tubes and tubular support structure of stainless steel or other corrosion resistant material.

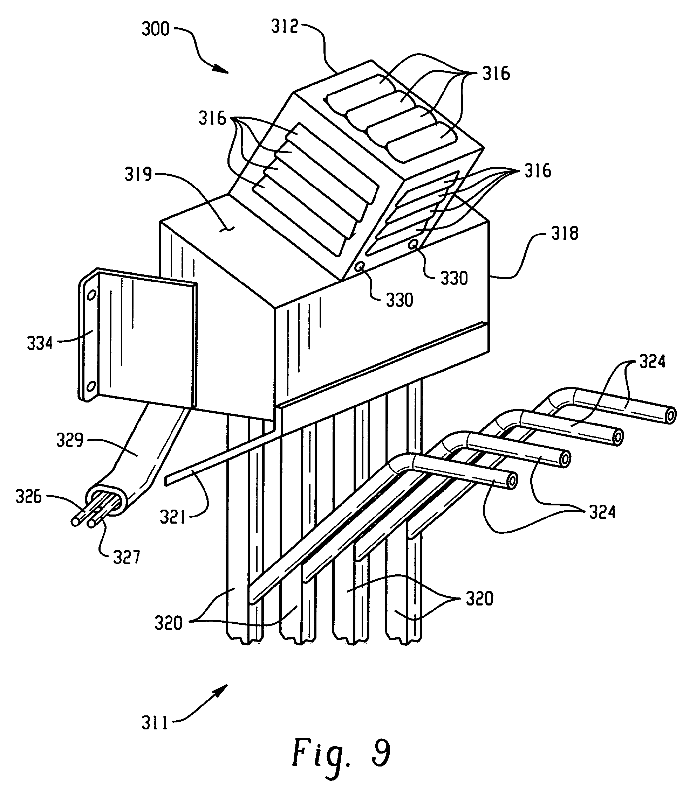

Referring to FIGURE 9, another version of the drying system of the present disclosure is indicated generally at 300 which is intended for spraying of sterilizing liquid only with electrical power externally switched off or disconnected. The system 300 has a rack indicated generally at 311 extending downwardly from the undersurface of an air manifold 318 which has a sloped upper surface 319 upon which is mounted a blower housing 312 for housing an unshown blower. The sloping upper surface 319 of the air manifold 318 is intended to have an air flow aperture, although unshown, similar to the aperture 15 of FIGURE 3 to permit forced air from blower housing 312 to enter the manifold 318.

The blower housing 312 has its inlet provided by a plurality of louvered vents 316 provided on the sides and upper surface of the blower housing. The manifold is adapted for attachment to a vertical surface by the brackets, one of which is shown at 334. One or more of the brackets may have surfaces thereon adapted for wall mounting. The rack 311 comprises a plurality of vertically downwardly extending riser tubes 320, each of which has a boot/glove hanging tube 324 cantilevered therefrom. The manifold is provided with power through cord 329 having therein electrical leads 326, 327, it being understood that the entrance of the power cord 329 to the manifold is sealed to prevent water penetration.

An optional splash shield 321 is angled downwardly from the front edge of the manifold and has cutouts for the risers 320 such that the splash shield can extend behind the risers.

At least one and preferably a plurality of drain holes 31 are provided in the front face of the blower housing 312 at the lowest edge thereof for permitting any liquid entering the blower housing to drain to the exterior. Thus, the version of FIGURE 9 is somewhat simpler in construction inasmuch as the interior drain tubes from the blower housing to the risers are omitted and the rearward overhang is eliminated. However, the version of FIGURE 9 is intended for use only where electrical power to the power cord 329 is disconnected exteriorly during the spraying of liquid sterilizer/disinfectant on the articles hanging on the rack. In another version intended for sterilizing with power connected, a conduit in the manifold drains any liquid in the blower housing directly into the rack or distribution tubes.

Although the system 300 of FIGURE 9 is illustrated as wall mounted, it would be understood that the system may also be configured as free standing in a manner similar to the versions shown in FIGURES 4 and 5.

The method of making a forced air drying system comprises providing a rack with a plurality of spaced tubular members adapted for receiving thereon the boots and/or gloves to be dried, providing an air manifold and connecting the manifold to the tubular members, disposing a blower housing on the manifold and sloping all exterior horizontal surfaces on the manifold and blower housing in order to prevent the pooling of sterilizer or disinfectant.

The step of connecting the manifold can include connecting the manifold to a plurality of vertically downwardly extending distribution tubes and providing hanging tubes extending from the distribution tubes. This could include extending the hanging tubes in cantilever from the distribution tubes. Further, removable air flow chokes can be provided in the free end of the hanging tubes and air flow in the rack can thus be balanced.

The step of providing a rack may include providing a rack having a member attached to the tubular members and adapted for wall mounting. The step of providing a manifold can include providing a manifold adapted for wall mounting. Alternatively, this step may include connecting a support structure to the manifold and rack and providing a free standing arrangement, which can include disposing rollers on the support structure.

The step of disposing a blower housing can include providing a plurality of louvered air inlets in the housing and further providing a sloped splash shield for the air inlets. This step can further include providing a rearward portion which is arranged to overhang the air manifold to form an updraft air inlet in the rearward overhanging portion.

The present disclosure thus describes a forced air drying system for drying multiple pairs of boots and/or gloves for food processing or other applications where it is required to spray the boots and/or gloves with liquid disinfectant or sterilizing agent prior to or during drying operations while in position on the hanging tubes of the rack. The blower housing, air manifold and distribution tubes are configured such that any horizontal or non-vertical surfaces which could result in pooling or trapping of disinfectant or sterilizer either externally on or in conjunction with drain holes interiorly are eliminated in any of the members of the system. One version is intended for use with electrical power connected and another lower cost version is intended for use with electrical power externally disconnected. The systems in either version may be wall mounted or arranged as free standing single sided or double sided rack portable drying systems.

The exemplary embodiments have been described with reference to the drawings. Obviously, modifications and alterations will occur to others upon reading and understanding the preceding detailed description. It is intended that the exemplary embodiment be construed as including all such modifications and alterations insofar as they come within the scope of the appended claims or the equivalents thereof.

## Claims

1. A forced air drying system (10,100, 200, 300) for boots and/or gloves for use in food processing, requiring spraying with a sterilizer or disinfectant; **characterised by**
(a) a rack (11, 111, 211, 311) with a plurality of spaced tubular members adapted for receiving thereon the boots and/or gloves to be dried;
(b) a manifold (18, 102, 202, 318) connected to the tubular members; and,
(c) a blower housing (12, 106, 206, 312) mounted on the manifold, the blower housing including an air inlet (16, 316), wherein any horizontal exterior surfaces of the blower housing, manifold and tubular members are angled or sloped to prevent pooling of the sterilizer or disinfectant.

2. The drying system as claimed in claim 1, **characterised in that** the tubular members include a plurality of spaced parallel distribution tubes (20, 130, 132, 134, 136, 230, 232, 234, 236, 320), each with at least one hanging tube (24, 148, 150, 152, 154, 324) cantilevered therefrom.

3. The drying system as claimed in claim 2, **characterised in that** the distribution tubes (20) are interconnected by a bracket (32) distal the manifold.

4. The drying system as claimed in any preceding claim, **characterised in that** the blower housing (12, 106, 206) includes a drain tube (36) which passes through the manifold, arranged to drain fluid into one of the tubular members (20, 130, 132, 134, 136, 230, 232, 234, 236, 320) of the rack (11, 111, 211, 311).

5. The drying system as claimed in claim 4, **characterised in that** the manifold includes a sloped upper surface (19, 104, 204) with the blower housing (12, 106, 206) mounted thereon, and the sloped upper surface includes a drain hole (17) communicating with the drain tube (36) with the drain tube.

6. The drying system as claimed in claims 2 and 5, **characterised in that** each of the distribution tubes (20) extends downwardly from the manifold, and has a drain hole (31) at the end remote from the manifold, and the manifold may include at least one drain tube (36) communicating at one end thereof with the blower housing drain hole (17), and at another end with one of the distribution tubes (20) of the rack.

7. The drying system as claimed in claim 1, **characterised in that** the tubular members include a plurality of distribution tubes (20) extending downwardly from the manifold, with a sloped bracket (32) interconnecting the end of the tubes remote from the manifold, and **in that** the ends (30) of the distribution tubes may be sloped with a drain hole (31) in the lower edge.

8. The drying system as claimed in claim 1, **characterised in that** the tubular members include a plurality of distribution tubes (130, 132, 134, 136, 230, 232, 234, 236) extending downwardly from the manifold, with the ends thereof remote from the manifold sloped, with a bottom bracket (138, 238) attached thereto closing the sloped ends; and the lowest edge of the sloped end of the tube may include a drain hole (140, 142, 144, 146, 240) therein.

9. The drying system as claimed in claim 2, **characterised in that** each cantilevered hanging tube (24, 148, 150, 152, 254, 324) includes a removable airflow choke (25) disposed in the free end thereof.

10. The drying system as claimed in claim 1, **characterised by** further comprising a sloping splash guard (21) for preventing sprayed sterilizer or disinfectant from entering the air inlet.

11. The drying system as claimed in claim 1, **characterised in that** the air inlet includes an updraft air passage on the blower housing.

12. The drying system as claimed in claim 1, **characterised in that** the blower housing (12, 106, 206) includes at least one drain hole (17) connected to a drain tube (36) passing through the manifold, and the blower housing (12, 106, 206) may include electrical leads (26, 27, 29, 326, 327, 329) sealed against liquid penetration.

13. The drying system as claimed in claim 1, **characterised in that** the tubular members include a plurality of distribution tubes (20, 130, 132, 134, 136, 230, 232, 234, 236, 320) extending downwardly from the manifold, the distribution tubes having a cross-section selected from one of (a) square and (b) rectangular.

14. The drying system as claimed in claim 1, **characterised in that** the blower housing (12, 106, 206, 312), manifold (18, 102, 202, 318) and tubular members are formed of one of (a) stainless steel and (b) corrosion resistant material.

15. The drying system as claimed in claim 1, **characterised by** further comprising a support structure (218, 220) connected to the manifold and rack for maintaining the rack in a free standing position, and in that the support structure may include rollers (126, 128, 246, 248, 250, 252) for portability.

16. The drying system as claimed in claim 15, **characterised in that** the rack (211) includes distribution tubes with cantilevered hanging tubes (242, 244) on opposite sides thereof.

## Patentansprüche

1. Gebläselufttrocknungssystem (10, 100, 200, 300) für Schuhe und/oder Handschuhe für den Einsatz in der Lebensmittelverarbeitung, welches das Aufsprühen eines Sterilisierungsmittels oder eines Desinfektionsmittels erfordert; **gekennzeichnet durch**
(a) ein Gestell (11, 111, 211, 311) mit mehreren beabstandeten rohrförmigen Elementen, welche für die Aufnahme der zu trocknenden Schuhe und/oder Handschuhe angepasst sind;
(b) einen Verteiler (18, 102, 202, 318), welcher mit den rohrförmigen Elementen verbunden ist; und
(c) ein auf dem Verteiler montiertes Gebläsegehäuse (12, 106, 206, 312), welches einen Lufteinlass (16, 316) aufweist, wobei jede der horizontalen Außenflächen des Gebläsegehäuses, des Verteilers und der rohrförmigen Elemente abgewinkelt oder schräg ist, um ein Ansammeln des Sterilisierungsmittels oder des Desinfektionsmittels zu verhindern.

2. Trocknungssystem nach Anspruch 1, **dadurch gekennzeichnet, dass** die rohrförmigen Elemente mehrere parallel beabstandete Verteilungsrohre (20, 130, 132, 134, 136, 230, 232, 234, 236, 320) aufweisen, jedes mit mindestens einem Hängerohr (24, 148, 150, 152, 154, 324), welches daran auskragend angeordnet ist.

3. Trocknungssystem nach Anspruch 2, **dadurch gekennzeichnet, dass** die Verteilungsrohre (20) durch einen von dem Verteiler distal gelegenen Haltebügel (32) miteinander verbunden sind.

4. Trocknungssystem nach einem der vorhergehenden Ansprüche, **dadurch gekennzeichnet, dass** das Gebläsegehäuse (12, 106, 206) ein Ablassrohr (36) aufweist, welches den Verteiler durchläuft, angeordnet zum Ablassen von Fluid in eines der rohrförmigen Elemente (20, 130, 132, 134, 136, 230, 232, 234, 236, 320) des Gestells (11, 111, 211, 311).

5. Trocknungssystem nach Anspruch 4, **dadurch gekennzeichnet, dass** der Verteiler eine schräge Oberseite (19, 104, 204) aufweist, mit dem Gebläsegehäuse (12, 106, 206) darauf montiert, und dass die schräge Oberseite ein Ablassloch (17) aufweist, welches mit dem Ablassrohr (36) in Verbindung steht.

6. Trocknungssystem nach Anspruch 2 und 5, **dadurch gekennzeichnet, dass** sich jedes der Verteilungsrohre (20) abwärts von dem Verteiler erstreckt und ein Ablassloch (31) an dem von dem Verteiler entfernten Ende aufweist, und dass der Verteiler mindestens ein Ablassloch (36), welches an einem Ende mit dem Ablassloch (17) des Gebläsegehäuses in Verbindung steht, und an dem anderen Ende mit einem der Verteilungsrohre (20) des Gestells aufweisen kann.

7. Trocknungssystem nach Anspruch 1, **dadurch gekennzeichnet, dass** die rohrförmigen Elemente mehrere Verteilungsrohre (20) aufweisen, welche sich abwärts von dem Verteiler erstrecken, mit einem schrägen Haltebügel (32), welcher das von dem Verteiler entfernte Ende der Rohre miteinander verbindet, und dass die Enden (30) der Verteilungsrohre abgeschrägt mit einem Ablassloch (31) am unteren Ende sein können.

8. Trocknungssystem nach Anspruch 1, **dadurch gekennzeichnet, dass** die rohrförmigen Elemente mehrere Verteilungsrohre (130, 132, 134, 136, 230, 232, 234, 236) aufweisen, welche sich abwärts von dem Verteiler erstrecken, mit ihren Enden entfernt von dem abgeschrägten Verteiler, mit einem daran angeschlossenen unteren Haltebügel (138, 238), welcher die schrägen Enden schließt; und dass der unterste Rand des schrägen Endes des Rohres ein Ablassloch (140, 142, 144, 146, 240) darin aufweisen kann.

9. Trocknungssystem nach Anspruch 2, **dadurch gekennzeichnet, dass** jedes auskragende Hängerohr (24, 148, 150, 152, 254, 324) eine abnehmbare Luftstromdrossel (25) aufweist, welche an dem freien Ende davon angeordnet ist.

10. Trocknungssystem nach Anspruch 1, **dadurch gekennzeichnet, dass** es ferner ein schräges Sprühschutzblech (21) zum Verhindern des Eintretens des gesprühten Sterilisierungsmittels oder Desinfektionsmittels in den Lufteinlass aufweist.

11. Trocknungssystem nach Anspruch 1, **dadurch gekennzeichnet, dass** der Lufteinlass einen Aufstrom-Luftdurchlass an dem Gebläsegehäuse aufweist.

12. Trocknungssystem nach Anspruch 1, **dadurch gekennzeichnet, dass** das Gebläsegehäuse (12, 106, 206) mindestens ein Ablassloch (17) aufweist, welches an ein den Verteiler durchlaufendes Ablassrohr (36) angeschlossen ist, und dass das Gebläsegehäuse (12, 106, 206) elektrische Leitungen (26, 27, 29, 326, 327, 329) aufweisen kann, welche gegen Flüssigkeitseindringen abgedichtet sind.

13. Trocknungssystem nach Anspruch 1, **dadurch gekennzeichnet, dass** die rohrförmigen Elemente mehrere Verteilungsrohre (20, 130, 132, 134, 136, 230, 232, 234, 236, 320) aufweisen, welche sich abwärts von dem Verteiler erstrecken, wobei die Verteilungsrohre einen Querschnitt aufweisen, welcher aus einem (a) quadratischen oder (b) rechtwinkligen ausgewählt ist.

14. Trocknungssystem nach Anspruch 1, **dadurch gekennzeichnet, dass** das Gebläsegehäuse (12, 106, 206, 312), der Verteiler (18, 102, 202, 318) und die rohrförmigen Elemente aus einem (a) rostfreiem Stahl oder (b) korrosionsbeständigem Material gebildet sind.

15. Trocknungssystem nach Anspruch 1, **dadurch gekennzeichnet, dass** es ferner eine tragende Struktur (218, 220) aufweist, welche an den Verteiler und das Gestell zum Halten des Gestells in einer stehenden Position angeschlossen ist, und dass die tragende Struktur Rollen (126, 128, 246, 248, 250, 252) zur Transportierbarkeit aufweisen kann.

16. Trocknungssystem nach Anspruch 15, **dadurch gekennzeichnet, dass** das Gestell (211) Verteilungsrohre mit auskragenden Hängerohren (242, 244) auf gegenüberliegenden Seiten davon aufweist.

## Revendications

1. Système de séchage par air forcé (10,100, 200, 300) pour des bottes et/ou des gants destinés à une utilisation dans la transformation des aliments, exigeant une pulvérisation d'un stérilisant ou désinfectant :
**caractérisé par**
(a) une grille (11, 111, 211, 311) avec une pluralité d'éléments tubulaires espacés adaptés pour recevoir au-dessus les bottes et/ou les gants à sécher ;
(b) un collecteur (18, 102, 202, 318) relié aux éléments tubulaires ; et,
(c) un logement de souffleur (12, 106, 206, 312) monté sur le collecteur, le logement de souffleur comportant une entrée d'air (16, 316), dans laquelle les surfaces extérieures horizontales quelconques du logement de souffleur, du collecteur et des éléments tubulaires présentent des angles et des pentes afin d'empêcher une accumulation du stérilisant ou du désinfectant.

2. Système de séchage selon la revendication 1, **caractérisé en ce que** les éléments tubulaires comportent une pluralité de tubes de distribution espacés parallèlement (20, 130, 132, 134, 136, 230, 232, 234, 236, 320), chacun ayant au moins un tube en suspension (24, 148, 150, 152, 154, 324) en porte-à-faux à partir de ceux-ci.

3. Système de séchage selon la revendication 2, **caractérisé en ce que** les tubes de distribution (20) sont interconnectés par un support (32) de manière distale par rapport au collecteur.

4. Système de séchage selon une quelconque des revendications précédentes, **caractérisé en ce que** le logement de souffleur (12, 106, 206) comporte un tube d'écoulement (36) qui traverse le collecteur, est agencé pour faire s'écouler le fluide dans l'un des éléments tubulaires (20, 130, 132, 134, 136, 230, 232, 234, 236, 320) de la grille (11, 111, 211, 311).

5. Système de séchage selon la revendication 4, **caractérisé en ce que** le collecteur comporte une surface supérieure en pente (19, 104, 204) avec le logement de souffleur (12, 106, 206) monté au-dessus, et la surface supérieure en pente comporte un orifice d'écoulement (1 7) communicant avec le tube d'écoulement (36).

6. Système de séchage selon les revendications 2 et 5, **caractérisé en ce que** chacun des tubes de distribution (20) s'étend vers le bas à partir du collecteur, et présente un orifice d'écoulement (31) à l'extrémité éloignée du collecteur, et le collecteur peut comporter au moins un tube d'écoulement (36) communicant, au niveau d'une extrémité de celui-ci, avec l'orifice d'écoulement (17) du logement de souffleur, et au niveau d'une autre extrémité avec un des tubes de distribution (20) de la grille.

7. Système de séchage selon la revendication 1, **caractérisé en ce que** les éléments tubulaires comportent une pluralité de tubes de distribution (20) s'étendant vers le bas du collecteur, avec un support en pente (32) interconnectant l'extrémité des tubes éloignée du collecteur, et **en ce que** les extrémités (30) des tubes de distribution peuvent être en pente avec un orifice d'écoulement (31) dans le bord inférieur.

8. Système de séchage selon la revendication 1, **caractérisé en ce que** the éléments tubulaires comportent une pluralité de tubes de distribution (130, 132, 134, 136, 230, 232, 234, 236) s'étendant vers le bas du collecteur, avec les extrémités de ceux-ci éloignées du collecteur en pente, avec un support de fond (138, 238) fixé à celui-ci, fermant les extrémités en pente; et le bord le plus inférieur de l'extrémité en pente du tube peut comporter un orifice d'écoulement (140, 142, 144, 146, 240) à l'intérieur.

9. Système de séchage selon la revendication 2, **caractérisé en ce que** chaque tube en suspension en porte-à-faux (24, 148, 150, 152, 254, 324) comporte un bouchon d'écoulement d'air amovible (25) disposé dans l'extrémité libre de celui-ci.

10. Système de séchage selon la revendication 1, **caractérisé en ce qu'**il comprend en outre un pare-éclaboussures en pente (21) pour empêcher que le stérilisant ou le désinfectant n'entre dans l'entrée d'air.

11. Système de séchage selon la revendication 1, **caractérisé en ce que** l'entrée d'air comporte un passage d'air ascendant sur le logement de souffleur.

12. Système de séchage selon la revendication 1, **caractérisé en ce que** le logement de souffleur (12, 106, 206) comporte au moins un orifice d'écoulement (17) relié à un tube d'écoulement(36) traversant le collecteur, et le logement de souffleur (12, 106, 206) peut comporter des fils de sortie (26, 27, 29, 326, 327, 329) rendus étanche contre toute pénétration de liquide.

13. Système de séchage selon la revendication 1, **caractérisé en ce que** les éléments tubulaires comportent une pluralité de tubes de distribution (20, 130, 132, 134, 136, 230, 232, 234, 236, 320) s'étendant vers le bas du collecteur, les tubes de distribution présentant une coupe transversale choisie entre un (a) carré et un (b) rectangle.

14. Système de séchage selon la revendication 1, **caractérisé en ce que** le logement de souffleur (12, 106, 206, 312), le collecteur (18, 102, 202, 318) et les éléments tubulaires sont formés d'un matériau parmi (a) l'acier inoxydable et (b) un matériau résistant à la corrosion.

15. Système de séchage selon la revendication 1, **caractérisé en ce qu'**il comprend en outre une structure support (218, 220) reliée au collecteur et à la grille pour maintenir la grille dans une position autoportante, et **en ce que** la structure support peut comporter des rouleaux (126, 128, 246, 248, 250, 252) pour sa mobilité.

16. Système de séchage selon la revendication 15, **caractérisé en ce que** la grille (211) comporte des tubes de distribution avec des tubes en suspension en porte-à-faux (242, 244) sur les côtés opposés de celle-ci.
